# FASCICULE DE BREVET EUROPEEN

(11) **EP 1 996 725 B1**
(45) Date de publication et mention de la délivrance du brevet: **18.05.2011**
(21) Numéro de dépôt: 07731073.8
(22) Date de dépôt: 02.03.2007
(51) Int. Cl.: C12Q 1/68

(54) **MÉTHODE DE DÉTECTION ÉLECTROCHIMIQUE DE SÉQUENCES CIBLES D'ACIDE NUCLÉIQUE**
VERFAHREN ZUM ELEKTROCHEMISCHEN NACHWEIS VON ZIELNUKLEINSÄURESEQUENZEN
METHOD FOR THE ELECTROCHEMICAL DETECTION OF TARGET NUCLEIC ACID SEQUENCES

(30) Priorité: 03.03.2006 FR 0601936; 10.01.2007 FR 0700157
(43) Date de publication de la demande: 03.12.2008
(73) Titulaire: Université Paris Diderot - Paris 7, 75205 Paris Cedex 13 (FR); UNIVERSITE DE BOURGOGNE, 21078 Dijon Cedex (FR); Centre National de la Recherche Scientifique, 75794 Paris Cedex 16 (FR)
(72) Inventeur: MARCHAL, Damien, 75009 Paris (FR); LIMOGES, Benoît, F-91220 Bretigny-sur-Orge (FR); DEQUAIRE, Murielle, 21000 Dijon (FR)
(74) Mandataire: Bertrand, Didier
(86) Numéro de dépôt international: PCT/FR2007/000373
(87) Numéro de publication internationale: WO 2007/099236

(56) Documents cités:
- EP-A- 1 500 933
- US-A1- 2002 106 683

## Description

La présente invention se rapporte à une méthode de détection électrochimique de séquences cibles d'acide nucléique et à un ensemble de détection pour la mise en oeuvre de ladite méthode.

Des méthodes connues permettent déjà grâce à une détection électrochimique d'identifier la présence ou non d'une séquence cible d'acide nucléique d'un échantillon biologique déterminé.

Ces méthodes permettent notamment de détecter dans un acide nucléique une séquence cible correspondant par exemple à une partie du patrimoine génétique d'un virus donné.

Selon les techniques connues, une fois cette séquence de nucléotides identifiée, on réalise des sondes formées d'oligonucléotides incorporant ladite séquence, puis on fixe ces sondes sur un support solide. Les sondes incluent un nombre de nucléotides donné qui présente une base nucléotidique oxydable. Ensuite, on met en contact l'échantillon biologique déterminé avec le support solide sur lequel sont greffés lesdites sondes de manière à ce que, le cas échéant, se produise l'hybridation de l'acide nucléique renfermant la séquence cible avec la sonde. Puis on fait réagir l'acide nucléique hybridé avec un complexe d'un métal de transition capable d'oxyder les bases des nucléotides de la sonde d'oligonucléotides; et on détermine la présence ou non de cette hybridation, qui se produit si l'échantillon biologique comporte un acide nucléique incluant ladite séquence cible, en appliquant un champ électrique variable à l'échantillon et en mesurant parallèlement le courant électrique qui y circule. Le courant électrique étant alors une fonction du nombre de bases données susceptibles d'être oxydées. En effet, lorsque l'on réalise un balayage en potentiel et que parallèlement on enregistre le courant électrique qui circule dans l'échantillon, la réponse en courant électrique est différente selon que la sonde est hybridée avec l'acide nucléique renfermant la séquence cible ou selon qu'elle ne l'est pas. En toute hypothèse le courant électrique d'oxydation de la base des nucléotides de la sonde hybridée a une valeur plus élevée que celui associé à l'oxydation de la base des nucléotides non hybridés.

On pourra notamment se référer au document US 2002/106 683, lequel décrit un tel procédé de détection.

Toutefois, une telle méthode est relativement complexe à mettre en oeuvre, car il est notamment nécessaire de préparer des sondes d'oligonucléotides afin de les greffer sur le support solide ce qui est long et coûteux.

On pourra également se référer au document EP-A-1 500 933, lequel divulgue la mise en oeuvre, selon une première étape, d'une méthode d'amplification de la séquence cible éventuellement présente dans l'échantillon biologique et ensuite, selon une seconde étape, d'une méthode de détection électrochimique.

Aussi, un problème qui se pose et que vise à résoudre la présente invention, est de fournir une méthode qui non seulement soit plus aisée à mettre en oeuvre mais aussi qui soit plus économique.

Dans le but de résoudre ce problème, et selon un premier aspect, la présente invention propose une méthode de détection électrochimique de séquences cibles d'acide nucléique, ladite méthode étant du type selon laquelle : on fournit un échantillon biologique susceptible de renfermer au moins un acide nucléique, ledit acide nucléique étant susceptible de contenir une séquence cible déterminée, ledit échantillon biologique étant mélangé à un agent oxydant, ladite séquence cible comportant une base nucléotidique apte à être oxydée par ledit agent oxydant ; on fournit des moyens complémentaires susceptibles de se coupler avec ladite séquence cible déterminée ; on applique un champ électrique audit échantillon, ledit champ électrique étant adapté à provoquer une réaction dudit agent oxydant avec ladite base nucléotidique, et on mesure un courant électrique qui traverse ledit échantillon pour déterminer la présence de ladite séquence cible ; selon l'invention lesdits moyens complémentaires comprennent des moyens d'amplification activables adaptés à répliquer ladite séquence cible, lesdits moyens d'amplification comprenant au moins des nucléotides d'un type incluant ladite base nucléotidique, lesdits nucléotides dudit type étant aptes à être consommés durant la réplication pour constituer des acides nucléiques répliqués, et ; on détermine la présence de ladite séquence cible déterminée si le courant électrique diminue lorsque lesdits moyens d'amplification sont activés.

Ainsi, une caractéristique de l'invention réside dans la mise en oeuvre d'une méthode d'amplification d'un acide nucléique et de la mesure simultanée d'un courant électrique, la mesure de ce courant électrique témoignant ou non de la consommation de l'un des éléments des moyens d'amplification durant celle-ci et précisément d'un des nucléotides libres. De la sorte, si la séquence cible d'acide nucléique correspond bien aux moyens d'amplification activables, cette séquence cible va être répliquée au cours du processus d'amplification, et le nombre de nucléotides libres utilisés comme substrat lors de cette réplication et dont la concentration est déterminée au départ, va décroître. En conséquence, si le nombre de nucléotides libres décroît au profit du nombre de nucléotides incorporés dans les acides nucléiques synthétisés durant l'amplification, l'agent oxydant ne pourra réagir qu'avec une quantité de plus en plus limitée de bases nucléotidiques correspondant aux nucléotides libres, et par conséquent moins de transferts électroniques se produiront, et le courant électrique finalement mesuré diminuera. On expliquera toutefois plus en détail dans la description détaillée qui suivra, que l'agent oxydant peut aussi oxyder les bases des nucléotides incorporés dans l'acide nucléique initialement présent et dans les acides nucléiques synthétisés par réplication, mais qu'en revanche le courant électrique qui en résulte est faible par rapport à celui qui résulte de l'oxydation des bases des nucléotides libres.

Selon un mode de mise en oeuvre de l'invention particulièrement avantageux, l'acide nucléique sur lequel on cherche à identifier une séquence cible est une molécule d'ADN ou d'ARN simple ou double brin. Aussi, les moyens d'amplification activables, comprennent des moyens adaptés à venir s'hybrider en amont d'une séquence cible et des moyens pour procéder à sa réplication. Le nombre d'acides nucléiques résultant de cette réplication croît exponentiellement avec le temps, de sorte que le nombre de nucléotides libres présentant la base nucléotidique oxydable baisse aussi exponentiellement avec le temps, et que parallèlement la diminution du courant qui traverse l'échantillon est rapidement perceptible.

Avantageusement, la base azotée qui est associée aux nucléotides libres est une base nucléotidique purique, par exemple la guanine ou un analogue chimique de la guanine, par exemple du type mercaptoguanine ou oxoguanine et l'agent oxydant utilisé est un complexe de ruthénium. Ce dernier présente une forme réduite et une forme oxydée, cette dernière venant catalyser de façon irréversible l'oxydation de la guanine. Lorsque le champ électrique est appliqué au mélange, le courant mesuré qui en résulte est sensiblement une fonction de la concentration en nucléotides libres présentant le résidu guanine, car bien que l'agent oxydant oxyde également la guanine des nucléotides incorporés dans l'acide nucléique répliqué, la cinétique d'oxydation ainsi que le coefficient de diffusion des nucléotides libres sont bien supérieurs à celui desdits acides nucléiques répliqués.

En outre, le nombre d'acides nucléiques résultant de la réplication desdites séquences cibles étant fonction du temps, la diminution du courant qui traverse l'échantillon est ainsi aussi fonction du temps. Ainsi si l'échantillon de départ présente une concentration importante en acide nucléique, et que ces acides nucléiques du même type présentent tous la séquence cible, la consommation en nucléotides, sera d'autant plus importante. Aussi, la mesure de la variation de courant électrique sera d'autant plus rapidement perceptible. En revanche, si la concentration en acide nucléique est moins importante, un temps plus important sera nécessaire pour observer la variation du courant. De ce fait, ladite invention est applicable pour une détermination quantitative de la séquence cible.

Aussi, de façon pratique, on appliquera un champ électrique et on mesurera le courant qui en résulte, par exemple au temps zéro puis régulièrement de manière à pouvoir enregistrer la diminution du courant électrique tout au long du processus d'amplification. Alternativement on pourra enregistrer le courant électrique après un temps précis déterminé.

En effet, a priori, la concentration en nucléotides libres présentant le résidu guanine est maximale avant d'avoir démarré l'amplification et par conséquent le courant électrique susceptible de traverser l'échantillon est alors lui aussi maximal. Ensuite, c'est en comparant la mesure du courant au cours ou après l'amplification, avec les mesures qui ont été réalisées durant le processus d'amplification, que l'on peut apprécier la différence. Si au cours de ces mesures une diminution significative du courant électrique est observée, cela signifie que les nucléotides libres présentant le résidu guanine ont été consommés, et donc que l'acide nucléique présentant la séquence cible est bien présent dans l'échantillon. On expliquera plus en détail dans la description détaillée, les vérifications complémentaires à faire avant de conclure à la présence de la séquence cible.

Au surplus, on mesure ce courant électrique après avoir appliqué le champ électrique durant un temps prédéterminé relativement constant pour une même amplification. En effet, entre l'instant où le champ électrique est appliqué et l'instant où le courant électrique qui traverse alors l'échantillon est maximal, une phase transitoire s'instaure durant laquelle la mobilité des espèces chimiques ionisées présentes dans l'échantillon va croître jusqu'à un maximum. Aussi, pour offrir un maximum de sensibilité, il est préférable de mesurer le courant électrique lorsqu'il est maximal, soit après un temps prédéterminé comme on l'expliquera plus en détail ci-après. Quoi qu'il en soit, la mesure du courant électrique est réalisée au moyen de techniques électrochimiques connues du type voltampérométrie à balayage de potentiel pouvant être linéaire, cyclique, à impulsion ou encore du type à saut de potentiel telle que la chronoampérométrie.

En outre, et de façon préférentielle, on applique ledit champ électrique entre des électrodes aptes à être baignées dans ledit échantillon. Par exemple, on applique un champ électrique entre une électrode à base d'oxydes métalliques ou à base de carbone ou bien encore à base d'un métal noble et une électrode de référence plongeant toutes les deux dans l'échantillon, en prenant soin de présenter une surface active constante des électrodes pour chaque mesure de courant d'une même amplification pour être bien certains que la variation de courant soit la résultante de la diminution des porteurs de charges et non pas de la variation de surface. On pourra par exemple choisir une électrode d'un métal noble tel que l'or ou le platine.

Selon un autre mode de mise en oeuvre de l'invention particulièrement avantageux, ledit agent oxydant est confiné à la surface de l'une desdites électrodes, soit par l'intermédiaire d'un gel, d'une membrane, ou d'un film appliqué sur l'électrode et qui emprisonne précisément l'agent oxydant ; soit par l'intermédiaire d'un polymère sur lequel est couplé l'agent oxydant, l'ensemble étant adsorbé ou greffé sur la surface de l'une des électrodes.

Selon un autre aspect, la présente invention propose un ensemble de détection électrochimique de séquences cibles d'acide nucléique, ledit ensemble comprenant: des moyens pour recevoir un échantillon biologique susceptible de renfermer au moins un acide nucléique, ledit acide nucléique étant susceptible de contenir une séquence cible déterminée, ledit échantillon biologique étant mélangé à un agent oxydant, ladite séquence cible comportant une base nucléotidique apte à être oxydée par ledit agent oxydant; des moyens complémentaires susceptibles de se coupler avec ladite séquence cible déterminée ; des moyens pour appliquer un champ électrique audit échantillon, ledit champ électrique étant adapté à provoquer une réaction dudit agent oxydant avec ladite base nucléotidique, et des moyens de mesure d'un courant électrique qui traverse ledit échantillon pour déterminer la présence de ladite séquence cible ; selon l'invention lesdits moyens complémentaires comprennent des moyens d'amplification activables adaptés à répliquer ladite séquence cible, lesdits moyens d'amplification comprenant au moins des nucléotides d'un type incluant ladite base nucléotidique, lesdits nucléotides dudit type étant aptes à être consommés durant la réplication pour constituer des acide nucléiques répliqués, et ; lesdits moyens de mesure fournissent une valeur décroissante de courant électrique lorsque lesdits moyens d'amplification sont activés, si ledit acide nucléique contient ladite séquence cible déterminée.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est un schéma réactionnel montrant schématiquement des réactions chimiques et électrochimiques intervenant lors de la mise en oeuvre de la méthode selon l'invention;
- la Figure 2 est une vue schématique montrant un premier ensemble de mise en oeuvre de la méthode conformément à l'invention selon un premier mode de mise en oeuvre ;
- la Figure 3 est une vue schématique montrant un élément d'un dispositif de mise en oeuvre de la méthode conformément à l'invention et selon un second mode de mise en oeuvre ;
- la Figure 4 est un graphique représentant des courbes d'évolution d'un courant électrique en fonction d'un potentiel appliqué et illustrant la méthode conformément à l'invention ;
- la Figure 5, est un graphique montrant la diminution d'un courant électrique en fonction d'un processus d'amplification;
- la Figure 6 est une vue schématique d'un second ensemble de mises en oeuvre de la méthode conformément à l'invention.
- la Figure 7 est un graphique montrant une courbe voltampérométrique.

La méthode de détection électrochimique de séquences cibles d'acide nucléique selon l'invention, vise à combiner la mise en oeuvre d'une méthode d'amplification d'acide nucléique et d'une méthode électrochimique pour pouvoir suivre la diminution d'une espèce chimique particulière traduisant la présence de ladite séquence cible d'acide nucléique. La méthode d'amplification qui sera décrite ci-dessous est du type « PCR » pour : Polymerase chain reaction. Toutefois, toute autre méthode d'amplification pourrait être mise en oeuvre avec la même efficacité. On citera notamment les méthodes du type «LCR » pour : Ligase Chain Reaction; «SDA » pour Strand Displacement Amplification; «RCA » pour Rolling Circle Amplification; « NASBA » pour Nucleic Acid Sequence Based Assay or Amplification ; ou encore «HDA» pour Helicase-dependent isothermal DNA Amplification. Ces méthodes sont toutes désignées par leur acronyme anglais.

Le principe de la méthode de type «PCR », consiste à utiliser, de manière répétitive, l'une des propriétés des ADN polymérases, pour synthétiser par réplication à partir des deux brins complémentaires composants l'ADN et d'une paire d'amorces (« primer » en langue anglaise) deux nouveaux brins copies des deux brins initiaux, les amorces, étant des petits brins d'acide nucléique d'environ 20 bases, capables de s'hybrider de façon spécifique, grâce à la complémentarité des bases, sur chacun des deux brins d'ADN à répliquer. Bien évidemment, les amorces sont choisies en fonction d'une séquence cible à révéler sur un acide nucléique.

Outre les ADN polymérases, qui permettent après que l'amorce se soit hybridée sur un brin, de synthétiser à partir de cette amorce un brin complémentaire, il est fourni également des nucléotides, et en particulier les quatre nucléotides dGTP, dATP, dTTP et dCTP constituant de l'ADN (respectivement désoxy-guanine-tri-phosphate, désoxy-adénosine-tri-phosphate, désoxy-tyrosine-tri-phosphate et désoxy-cytosine-tri-phosphate), que l'ADN polymérase vient assembler pour former un brin complémentaire répliqué.

Par ailleurs, le milieu réactionnel dans lequel sont introduits : l'échantillon à tester susceptible de contenir la séquence cible d'acide nucléique, l'ADN polymérase, les amorces, et les quatre types de nucléotides et qui forment un mélange réactionnel, est bien évidemment liquide et tamponné. En outre, et selon la méthode, ce mélange réactionnel va être alternativement soumis à des variations de température, correspondant à différentes phases, de dénaturation, d'hybridation et d'élongation de l'acide nucléique, au cours d'un même cycle. De manière classique, le mélange réactionnel pourra subir successivement une trentaine de cycles.

Par ailleurs, et c'est là un objet de l'invention, la méthode d'amplification dont le principe a été décrit ci-dessus, va être couplée à des moyens électrochimiques, permettant de révéler, le cas échéant, la disparition des nucléotides de l'un des quatre types, au fur et à mesure des cycles d'amplification, les nucléotides dudit un des quatre types, étant incorporés dans les brins d'ADN complémentaires par action de l'ADN polymérase.

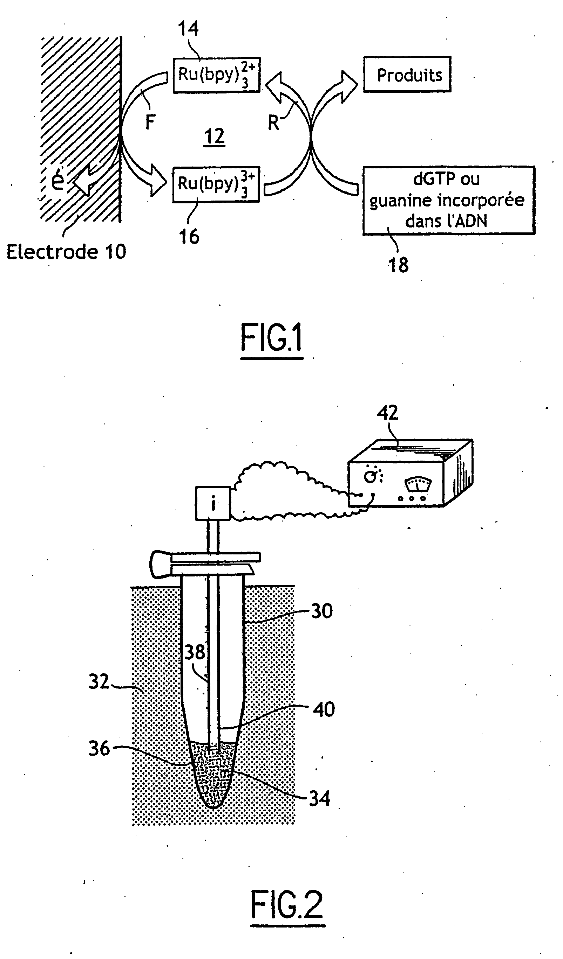

Pour ce faire, on utilise un agent oxydant et en particulier un complexe de ruthenium, par exemple le tris(2, 2'-bipyridyl)ruthenium(II), qui est susceptible, sous sa forme oxydée de venir oxyder à son tour la guanine des nucléotides présentant le résidu guanine : le dGTP. Bien évidemment, tout autre agent oxydant capable d'oxyder la guanine pourra être utilisé, on citera par exemple IrCl₆⁴⁻. Ainsi que l'illustre la Figure 1, sur laquelle est représentée une électrode 10 plongée dans un milieu réactionnel 12 aqueux, l'agent oxydant étant incorporé au mélange réactionnel précité, lorsqu'on applique un champ électrique à ce mélange, l'agent oxydant évolue tout d'abord de sa forme réduite 14 vers sa forme oxydée 16 selon la flèche F, en cédant un électron à l'électrode 10. Par ailleurs, sous sa forme oxydée 16, l'agent oxydant est susceptible, d'oxyder à son tour une guanine d'un nucléotide 18 présentant précisément un résidu guanine, et qui est soit libre ou soit insérée dans un acide nucléique. En oxydant cette guanine, l'agent oxydant est simultanément réduit selon la flèche R alors que la guanine définitivement oxydée n'intervient plus dans la réaction. Le courant mesuré à l'électrode, c'est-à-dire le flux électronique, est essentiellement dû au courant d'oxydation des nucléotides libres présentant un résidu guanine et pour une part infime aux nucléotides insérés dans un brin d'ADN. Une explication pouvant être donnée par la différence de coefficient de diffusion mais aussi la différence de cinétique d'oxydation des guanines entre un nucléotide présentant un résidu guanine libre et un nucléotide présentant un résidu guanine incorporé dans un acide nucléique.

En conséquence, si les nucléotides incluant la guanine sont consommés durant les cycles d'amplification, au même titre que les autres nucléotides, pour synthétiser les acides nucléiques répliqués qui statistiquement incluent quasiment autant de nucléotides des quatre types précités, le flux électronique et par conséquent le courant mesuré à l'électrode diminuera lui aussi par voie de conséquence.

Dans le but d'illustrer la méthode conforme à l'invention, on décrira ci-après un exemple de mise en oeuvre.

La Figure 2, illustre, selon un premier mode de mise en oeuvre, un tube 30 adapté à être installé dans un thermocycleur 32 qui lui, va permettre de porter le tube 30 à des températures prédéterminées, représentatives des différentes étapes de l'amplification selon la méthode du type «PCR ». De manière classique, dans une première étape d'un cycle, le tube est chauffé quelques secondes à 94° C pour provoquer la dénaturation de l'acide nucléique et ici de l'ADN. Ensuite dans une seconde étape d'environ une minute, la température est rapidement abaissée à 58° C de manière à provoquer l'hybridation des amorces. Puis, le tube est ensuite porté à une température de 72° C durant une minute également, de manière à activer l'ADN polymérase afin de provoquer l'élongation des brins complémentaires. Et ensuite, un nouveau cycle est entamé.

Le mélange réactionnel 34 est logé dans le culot 36 du tube 30 et deux électrodes 38, 40 introduites dans le tube 30 plongent dans le mélange réactionnel 34. L'une des électrodes, 38, est une électrode en carbone par exemple, tandis que l'autre électrode 40 est l'électrode de référence. Ces électrodes 38, 40 sont respectivement connectées à un potentiostat 42 permettant de faire varier un potentiel électrique selon un profil déterminé, entre les deux électrodes, et d'enregistrer parallèlement le courant électrique qui les traverse.

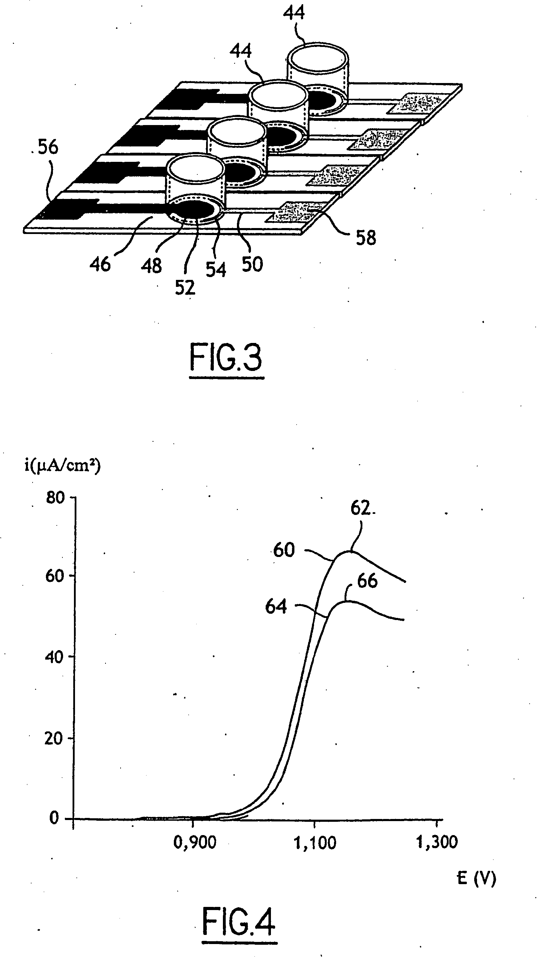

Selon un second mode de mise en oeuvre dont on a reproduit les éléments essentiels sur la figure 3, la méthode d'amplification est en tous points identiques à la méthode précitée, cependant, la détection n'est plus réalisée durant l'amplification mais à l'issue de celle-ci. Dans ce cas, aucune électrode n'est directement introduite dans le tube 30, mais le mélange réactionnel 34 est lui introduit dans une ou plusieurs micro-cuvettes 44 illustrées sur la Figure 3. Ces micro-cuvettes 44 sont scellées de façon étanche sur une plaque de type « circuit intégré » 46 sur laquelle ont été sérigraphiées des électrodes 48, 50. Ces électrodes 48, 50 présentent respectivement une extrémité active 52, 54 située dans le fond des micro-cuvettes 44 et elles s'étendent en dehors respectivement vers des extrémités de connexion 56, 58. De la même façon que dans le premier mode de mise en oeuvre précité, les extrémités de connexion 56, 58 sont adaptées à être reliées à un potentiostat pour appliquer un champ électrique au mélange réactionnel situé dans la micro-cuvette 44.

On notera que ces micro-cuvettes 44 sont également adaptées à des méthodes d'amplification du type« NASBA », « RCA » ou « HDA » citées ci-dessus.

### Exemple d'application 1

On détaillera ci-après un exemple d'application de ladite méthode pour détecter la présence du Cytomégalovirus (CMV) dans un échantillon biologique. Le génome de ce virus présente une séquence bien identifiée de 406 paires de bases et on utilise les deux amorces commerciales AC1 et AC2 qui permettent l'amplification spécifique de cette séquence (références 60-003 et 60-004 de la société Argene). Le volume total du mélange réactionnel est égal à 50 µl et il contient les deux amorces à une concentration de 0,8 µmol/l, les nucléotides des quatre types à une concentration de 100 µmol/l, la polymérase à une concentration de 0,02 unité par µl, une concentration en complexe de ruthénium de 20 µmol/l et une dilution au dixième de tampon 10X. Bien évidemment, le mélange réactionnel contient un échantillon biologique et en l'espèce, un extrait cellulaire incorporant le Cytomégalovirus.

La mesure électrochimique s'effectue à l'aide du potentiostat 42 à travers l'application d'une technique voltampérométrique qui consiste à appliquer une variation de potentiel entre les deux électrodes 38, 40 ou 52, 54, en démarrant, par exemple, d'un potentiel initial de 0,5 V par rapport à une électrode au calomel, jusqu'à un potentiel final de 1,3 V (par rapport à la même électrode au calomel), pour une vitesse de balayage pouvant être compris entre 0,01 et 100 V par seconde. Parallèlement, le courant électrique résultant est enregistré au cours du balayage de potentiel.

On se référera à la Figure 4, sur laquelle sont reportées deux courbes voltampérométriques enregistrées à une vitesse de 0,1 V.s⁻¹ par le potentiostat en fonction de la différence de potentiel appliquée entre les électrodes précitées et représentée ici en densité de courant. Une première courbe 60 représente l'évolution du courant électrique mesuré au deuxième cycle d'amplification selon l'expérience précitée. L'échantillon biologique contenait initialement 100000 copies de la séquence cible. Cette courbe atteint un premier extremum 62 d'environ 68 µA par cm². Une deuxième courbe 64 représente l'évolution du courant électrique mesuré au trentième cycle selon l'expérience précitée. Cette courbe atteint un premier extremum 66 d'environ 56 µA par cm². Cet extremum 66 est situé sensiblement 12 µA par cm² en dessous du premier extremum 62.

Par ailleurs, on a bien vérifié au préalable, que les deux étapes du schéma opératoire précité, appliqué à un échantillon biologique ne contenant pas le Cytomégalovirus avec les moyens d'amplification précitée, conduisait à l'obtention de deux courbes sensiblement identiques. Par conséquent, lorsque le virus n'est pas présent dans l'échantillon biologique, l'amplification ne se produit pas.

Ainsi, il apparaît très clairement en comparant les deux courbes 60, 64 que l'intensité du courant électrique a diminué après 30 cycles d'amplification par rapport à sa valeur initiale avant amplification. Par conséquent, il apparaît clairement que la présence du Cytomégalovirus dans l'acide nucléique de l'échantillon biologique, a été reconnu par les amorces AC1 et AC2 correspondantes, et que l'élongation des brins complémentaires s'est bien produite en consommant les nucléotides et en particulier les nucléotides du type dGTP présentant le résidu guanine, puisque c'est la base de ce seul nucléotide que l'agent oxydant, le complexe de ruthénium, est susceptible d'oxyder. Or, la mesure du courant électrique est essentiellement le résultat de la mesure du flux d'électrons correspondant à la réduction du complexe de ruthénium et parallèlement à l'oxydation des nucléotides incluant la guanine dGTP. Aussi, lorsque ces derniers nucléotides disparaissent partiellement, car ils sont intégrés dans les brins d'acide nucléique complémentaires, leur quantité à l'état libre diminue et par conséquent le courant d'oxydation qui en résulte diminue aussi.

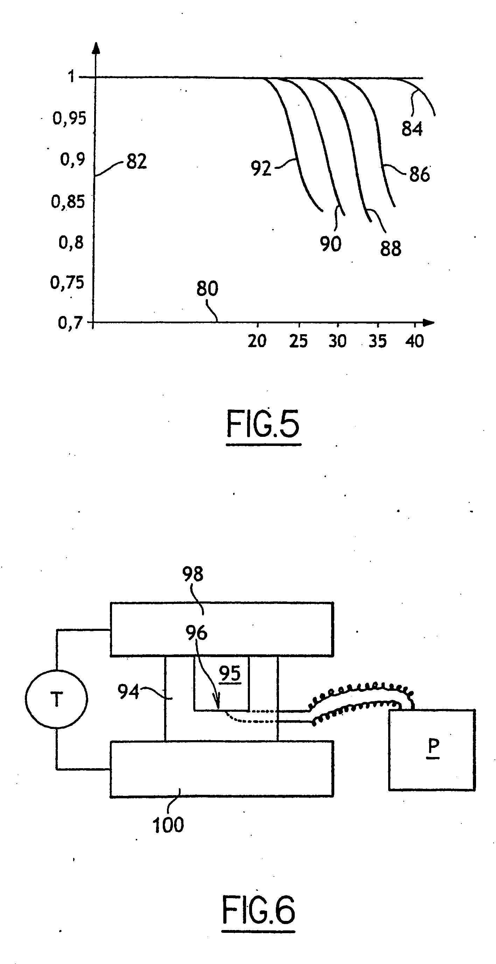

Par ailleurs, on se référera à présent au graphique illustré sur la Figure 5, pour décrire le principe de la quantification d'une séquence cible d'un acide nucléique dans un échantillon donné.

En abscisse 80 du graphique, sont portés les nombres de cycles de réplication et en ordonnée 82 sont reportées les valeurs normalisées des courants relevées à chaque cycle à un potentiel donné suivant le mode de mise en oeuvre illustré sur la Figure 2. Ces valeurs normalisées de courant correspondent aux mesures de courant divisées par la mesure du courant réalisée avant le démarrage de l'amplification.

Les cinq courbes représentées, 84, 86, 88, 90, 92 représentent les courbes tracées en partant d'un échantillon biologique comprenant respectivement, zéro copie de la séquence cible CMV, 84, 1000 copies de ladite séquence cible, 86, 10 000 copies de ladite séquence cible, 88, 100 000 copies de ladite séquence cible, 90, et 1 000 000 de copies de ladite séquence cible, 92.

Ainsi, on constate que, plus l'échantillon biologique contient d'acides nucléiques incorporant la séquence cible, plus le courant électrique qui traverse l'échantillon diminue rapidement en fonction du nombre de cycles. En effet, plus l'échantillon contient à l'origine d'acides nucléiques incorporant la séquence cible, plus la réplication va consommer de nucléotides du type dGTP et par conséquent plus le courant électrique va chuter tôt en fonction du nombre de cycles. De la sorte, on comprend qu'une mesure de la quantité d'acides nucléiques incorporant la séquence cible est possible, en déterminant le nombre de cycles à partir duquel, la quantité de courant qui traverse l'échantillon s'infléchit. En outre, cette Figure 5 montre également que la présence de la séquence cible est détectable même si seulement 1000 copies de ladite séquence cible sont initialement présentes dans l'échantillon.

Ainsi donc, la méthode selon l'invention appliquée à un échantillon biologique susceptible de contenir un virus identifié permet non seulement, grâce à la mise en oeuvre d'une méthode d'amplification et d'une mesure électrochimique de révéler la présence ou l'absence dudit virus, mais également de le quantifier. Par conséquent, en comparaison des méthodes mises en oeuvre selon l'art antérieur, pour lesquelles la préparation du matériel d'identification est complexe, selon la présente invention, il est seulement nécessaire de mettre en oeuvre une méthode d'amplification classique, puis durant l'amplification de soumettre l'échantillon biologique à un champ électrique et de mesurer le courant qui en résulte. Cette méthode utilise un agent oxydant qui n'est nullement mis en oeuvre dans les méthodes d'amplification classiques, pour révéler la disparition d'une espèce en l'occurrence ici un type de nucléotide présentant le résidu guanine ou un composé ayant le même rôle biologique que la guanine. En outre, le choix de l'agent oxydant dépendra de la nature du type de nucléotides dont on désire mesurer la disparition.

L'exemple de mise en oeuvre ci-dessus concerne un acide nucléique à double chaîne, soit une molécule d'ADN. Cependant, il peut tout à fait être appliqué à un acide nucléique monocaténaire de type ARN ou d'ADN, moyennant la mise en oeuvre de moyens d'amplification adaptés. Dans les deux cas, l'amplification de ces acides nucléiques entraîne la consommation de nucléotides incluant la guanine, et par conséquent la diminution de cette espèce dans le milieu au fur et à mesure du processus d'amplification.

Ainsi donc, quel que soit le mode de mise en oeuvre de la méthode objet de l'invention, soit le premier mode décrit ci-dessus à l'appui de la figure 2, soit le second mode décrit en référence à la figure 3, on évalue le moment à partir duquel la valeur du courant électrique chute de manière significative, en effectuant des mesures au cours du processus d'amplification. Cela permet non seulement de révéler la présence de la séquence cible d'ADN, mais également de remonter indirectement à la concentration initiale en acide nucléique incorporant la séquence cible à détecter. En effet, plus le nombre de copies initiales d'acide nucléique incorporant la séquence cible est important, plus le courant électrique diminuera tôt, et à l'inverse, plus le nombre de copies est faible et plus la chute de courant sera observée tardivement.

Par ailleurs, et selon un autre aspect illustré sur la Figure 6, la présente invention concerne également un ensemble spécialement adapté à la détection électrochimique de séquences cibles d'acides nucléiques. Cet ensemble comprend au moins une micro-cuvette 94 telle que représentée sur la Figure 3, pour recevoir un échantillon biologique 95, mais ici équipée d'une isolation thermique. Cette micro-cuvette 94 présente des électrodes sérigraphiées 96 dans le fond et qui sont reliées à un potensiostat P. Par ailleurs, la micro-cuvette 94 est placée entre deux modules à effet Peltier 98, 100 reliées par un générateur adapté à réguler la température à l'intérieur de la micro-cuvette 94. De la sorte, l'échantillon biologique 95, est susceptible d'être porté à des températures allant jusqu'à 94° C et en suivant des variations brutales tout comme la méthode précitée et de type PCR. De plus, à l'échantillon biologique est ajouté du matériel d'amplification activable et notamment des nucléotides présentant une base nucléotidique oxydable.

Ainsi, l'ensemble illustré sur la Figure 6, qui est susceptible d'être commandé par un ordinateur, est adapté à fournir automatiquement l'information selon laquelle, la séquence cible recherchée est bien contenue dans l'échantillon inséré dans la micro-cuvette 94, et si tel en est le cas, la concentration en acide nucléique comprenant la séquence cible.

L'ordinateur, est alors chargé par un programme d'ordinateur, capable de faire fonctionner simultanément les moyens de réplication, c'est-à-dire les modules à effet Peltier 98, 100 selon des cycles prédéfinis et le potentiostat entre les cycles pour mesurer la quantité de courant traversant l'échantillon 95 pour des valeurs de potentiel données.

En outre, lors de la mise en oeuvre des moyens d'amplification, l'état de surface de ladite électrode est modifié. Par ailleurs, au fil des cycles d'amplification, le solvant du mélange réactionnel, en l'espèce l'eau, est susceptible de s'évaporer compte tenu des températures appliquées. Toute évaporation du solvant provoque, par nature, une augmentation de la concentration des solutés.

En conséquence, la mesure du courant qui est proportionnelle à la concentration en base nucléotidique consommée pourrait ne pas être tout à fait corrélée à la quantité d'acide nucléique amplifié.

Aussi, un sous problème qui se pose alors, est de pouvoir s'affranchir à la fois des perturbations à l'électrode et de l'évaporation du solvant.

Pour ce faire, et selon encore un autre mode de mise en oeuvre de l'invention particulièrement avantageux, la méthode de détection comprend en outre les étapes suivantes : on fournit un composé redox apte à réagir à une valeur de champ électrique décalé différente de la valeur de champ électrique à laquelle ledit agent oxydant et ladite base nucléotidique réagissent ; on mesure la valeur du courant électrique redox à ladite valeur de champ électrique décalé ; et, on détermine la présence de ladite séquence cible déterminée si la différence de courant électrique entre le courant électrique redox et ledit courant électrique de réaction dudit agent oxydant et de ladite base nucléotidique diminue lorsque lesdits moyens d'amplification sont activés.

Ainsi, en choisissant un composé redox, ou étalon interne, par exemple un ferrocène, un viologène ou encore un complexe d'osmium, dont le potentiel d'oxydation est décalé par rapport au potentiel d'oxydation dudit agent oxydant et de ladite base nucléotidique, par exemple de 800 mV, l'oxydation du composé redox ne perturbe pas la mesure du courant d'oxydation de l'agent oxydant et de la base. Au surplus, cet agent oxydant n'interfère pas avec les moyens d'amplification activables:

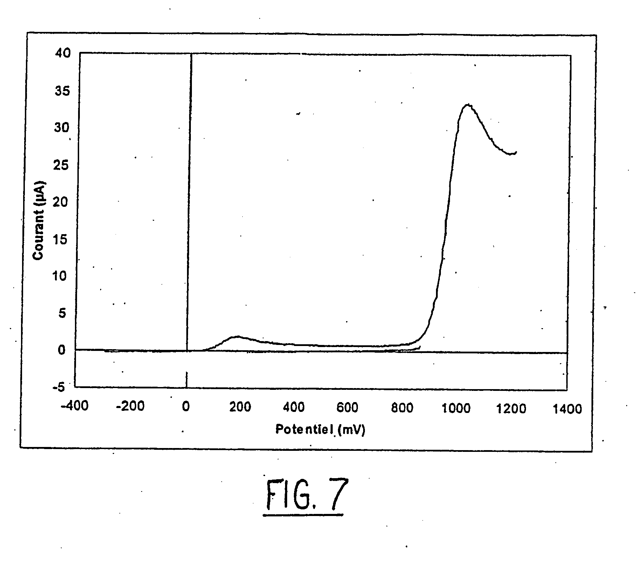

Selon un premier mode de réalisation conformément à cet autre mode de mise en oeuvre, pour lequel on pourra se référer à la Figure 7, on procède à la normalisation de la mesure du courant électrique pour un seul échantillon lors d'une même expérience.

Ainsi, on procède à la mise en oeuvre d'un échantillon dans un mélange réactionnel identique à celui de l'exemple d'application précité, excepté qu'on y incorpore en plus du ferrocenemethanol à une concentration de 50 µM et que l'on applique une variation de potentiel entre les deux électrodes, en démarrant à 50 mV pour terminer à 1300 mV. On obtient de la sorte, pour le premier cycle d'amplification, la courbe voltampérométrique reportée à la Figure 7.

Cette courbe présente non plus un seul extremum, mais deux. Un premier 210 situé vers 1100 mV par rapport à l'électrode au calomel saturé, et qui correspond aux extremums des courbes illustrées sur la Figure 4, et un second 220 situé vers 200 mV, lequel correspond à l'oxydation du ferrocenemethanol. Le premier extremum résulte de l'oxydation de l'agent oxydant, le complexe de ruthénium et la mesure du courant correspondant est sensiblement de 32 µA, tandis que le second qui résulte de l'oxydation du composé redox est d'environ 2 µA.

Après le balayage en potentiel entre 50 mV et 1300 mV et l'enregistrement simultané des courants d'intensité, on procède à la normalisation du courant correspondant au premier extremum 210, en divisant sa valeur, sur l'exemple illustré sur la Figure 7 : 32 µA, par la valeur du courant correspondant au second extremum 220, 2 µA et on obtient 16.

Ensuite, on procède de la même façon pour tous les cycles d'amplification de ladite expérience. De la sorte, la mesure de la variation de la différence des valeurs d'intensité des deux extremums 210, 220 résulte uniquement de la diminution de la concentration en base nucléotidique, en l'espèce les dGTP, et permet de s'affranchir des conditions de l'expérience.

Partant, et selon un second mode de réalisation on procède de manière identique pour une série d'échantillons respectivement introduit dans une série de micro-cuvettes du type de celles illustrées sur la Figure 3 de façon à réaliser une série d'expériences. Ces échantillons biologiques proviennent avantageusement d'une même source, et il s'agit là de confirmer la présence de la séquence cible déterminée. Aussi, en normalisant les valeurs d'intensité correspondant à l'oxydation de l'agent oxydant respectivement pour chacune des expériences, elles sont alors comparables entre elles puisqu'elles sont indépendantes des variations de volume et d'état de surface des électrodes.

Ainsi, selon cet autre mode de mise en oeuvre de l'invention, on affine la détection de la consommation réelle de nucléotides, ce qui permet de conclure à la présence d'une séquence cible après un faible nombre de cycles d'amplification. En conséquence, on diminue le temps nécessaire à l'établissement du diagnostic.

En outre, et ainsi que l'illustre le second mode de réalisation précité, des mesures peuvent être comparées quelles que soient les conditions opératoires.

### Exemple d'application 2

En outre, la méthode de détection selon l'invention, n'est pas seulement applicable aux virus, mais aussi aux bactéries. Ainsi, elle a été appliquée pour détecter la présence de bactéries, et en particulier . *Achromobacter xylosoxidans* dont des éléments génétiques mobiles et plus précisément les intégrons jouent un rôle fondamental dans l'acquisition de gènes de résistance aux antibiotiques.

Ainsi, trois fragments d'ADN caractéristiques de cette souche bactérienne ont été identifiés et amplifiés par des méthodes de type PCR. La mise en oeuvre de la méthode selon l'invention a permis de détecter la présence de la bactérie précitée en identifiant un premier gène « *ver-1* » comptant environ 900 paires de bases. Ce gène code pour une bêta lactamase qui confère un haut niveau de résistance à divers antibiotiques et notamment l'amoxicilline et la ticarcilline. Les moyens d'amplification activables, comportent les quatre nucléotides, de l'ADN polymérase et une paire d'amorces «VEB-F et VEB-R » spécifiques du gène, et ils ont été incorporés à un échantillon biologique contenant ladite bactérie.

Ainsi on observe, un voltamogramme similaire à celui qui est représenté sur la Figure 4. Selon les conditions de l'expérience, une première courbe correspondant à une mesure du courant électrique avant d'avoir démarré l'amplification, présente un extremum d'environ 6 µA pour une valeur de potentiel d'environ 1,075 V. Une seconde courbe réalisée après amplification montre un extremum diminué d'environ 1 µA. Par conséquent, la diminution du courant électrique résulte de la consommation en nucléotides lors de l'amplification et par conséquent de la présence effective du gène «*veb-1*».

Un deuxième fragment de 100 paires de bases, du gène *«int I* », caractéristiques de la bactérie précitée, a été identifié et détecter de la même façon.

Un troisième fragment de 2500 paires de bases, caractéristique de ladite bactérie, a aussi pu être détecté de façon analogue. La séquence nucléotidique de ce troisième fragment correspond à une région variable de l'intégron contenant l'ensemble des cassettes dont le gène «*veb-1* ».

Ainsi, la méthode objet de l'invention, peut être appliquée à des amplifications de fragments de matériel génétique de tailles différentes sur des protocoles différents et avec des moyens d'amplification activable ou quitte biologique différents.

## Revendications

1. Méthode de détection électrochimique de séquences cibles d'acide nucléique, ladite méthode étant du type selon laquelle :
- on fournit un échantillon biologique susceptible de renfermer au moins un acide nucléique, ledit acide nucléique étant susceptible de contenir une séquence cible déterminée,
- on fournit un agent oxydant apte à oxyder au moins une base nucléotidique de ladite séquence cible déterminée;
- on fournit des moyens complémentaires susceptibles de se coupler avec ladite séquence cible déterminée, lesdits moyens complémentaires comprenant des moyens d'amplification activables adaptés à répliquer ladite séquence cible lorsque ledit échantillon biologique est mélangé avec lesdits moyens d'amplification activables, lesdits moyens d'amplification comprenant au moins des nucléotides d'un type incluant ladite base nucléotidique, lesdits nucléotides dudit type étant aptes à être consommés durant la réplication pour constituer des acides nucléiques répliqués ;
- on mélange ledit agent oxydant audit échantillon biologique ;
- on applique un champ électrique audit échantillon, ledit champ électrique étant adapté à provoquer une réaction dudit agent oxydant avec ladite base nucléotidique ; et on mesure un courant électrique qui traverse ledit échantillon pour déterminer la présence de ladite séquence cible si le courant électrique diminue;
**caractérisée en ce qu'**elle comprend dans l'ordre les étapes suivantes :
a) on mélange ledit échantillon biologique et ledit agent oxydant avec lesdits moyens d'amplification activables ;
b) on active lesdits moyens d'amplification activables ; et,
c) on soumet l'échantillon biologique à un champ électrique durant l'amplification pour mesurer le courant qui en résulte, de manière à déterminer la présence de ladite séquence cible déterminée si le courant électrique diminue.

2. Méthode de détection selon la revendication 1, **caractérisée en ce que** ledit acide nucléique est une molécule d'ADN ou d'ARN.

3. Méthode de détection selon la revendication 1 ou 2, **caractérisée en ce que** ladite base nucléotidique est une base azotée purique.

4. Méthode de détection selon la revendication 3, **caractérisée en ce que** ladite base nucléotidique est la guanine ou un analogue chimique dé la guanine.

5. Méthode de détection selon l'une quelconque des revendications 1 à 4, **caractérisée en ce que** ledit agent oxydant est un complexe de ruthénium.

6. Méthode de détection selon l'une quelconque des revendications 1 à 5, **caractérisée en ce que**, lesdites séquences cibles étant répliquées au cours du temps, on mesure ledit courant électrique après avoir appliqué ledit champ électrique durant un temps déterminé.

7. Méthode de détection selon l'une quelconque des revendications 1 à 6, **caractérisée en ce qu'**on réplique ladite séquence cible selon des cycles d'amplification, et **en ce qu'**on mesure ledit courant après un nombre déterminé de cycle.

8. Méthode de détection selon l'une quelconque des revendications 1 à 7, **caractérisée en ce qu'**on applique ledit champ électrique entre des électrodes aptes à être baignées par ledit échantillon.

9. Méthode de détection selon la revendication 8, **caractérisée en ce que** l'une desdites électrodes est une électrode à base d'oxydes métalliques.

10. Méthode de détection selon la revendication 8, **caractérisée en ce que** l'une desdites électrodes est une électrode à base d'un métal noble.

11. Méthode de détection selon la revendication 8, **caractérisée en ce que** l'une desdites électrodes est une électrode de carbone.

12. Méthode de détection selon l'une quelconque des revendications 8 à 11, **caractérisée en ce que** ledit agent oxydant est confiné à la surface de l'une desdites électrodes.

13. Méthode de détection selon l'une quelconque des revendications 1 à 12, **caractérisée en ce qu'**elle comprend en outre les étapes suivantes :
- on fournit en outre un composé redox apte à réagir à une valeur de champ électrique décalé différente de la valeur de champ électrique à laquelle ledit agent oxydant et ladite base nucléotidique réagissent ;
- on mesure la valeur du courant électrique redox à ladite valeur de champ électrique décalé ; et,
- on détermine la présence de ladite séquence cible déterminée si la différence de courant électrique entre le courant électrique redox et ledit courant électrique de réaction dudit agent oxydant et de ladite base nucléotidique diminue lorsque lesdits moyens d'amplification sont activés.

14. Ensemble de détection électrochimique de séquences cibles d'acide nucléique, ledit ensemble comprenant :
- des moyens (30, 44, 94) pour recevoir un échantillon biologique (34, 95) susceptible de renfermer au moins un acide nucléique, ledit acide nucléique étant susceptible de contenir une séquence cible déterminée, ledit échantillon biologique étant mélangé à un agent oxydant, ladite séquence cible comportant au moins une base nucléotidique apte à être oxydée par ledit agent oxydant ;
- des moyens complémentaires susceptibles de se coupler avec ladite séquence cible déterminée ;
- des moyens (38, 42, 52, 54, 96) pour appliquer un champ électrique audit échantillon, ledit champ électrique étant adapté à provoquer une réaction dudit agent oxydant avec ladite base nucléotidique, et des moyens de mesure (42, P) d'un courant électrique qui traverse ledit échantillon pour déterminer la présence de ladite séquence cible ;
**caractérisée en ce que** lesdits moyens complémentaires comprennent des moyens d'amplification activables adaptés à répliquer ladite séquence cible, lesdits moyens d'amplification comprenant au moins des nucléotides d'un type incluant ladite base nucléotidique, lesdits nucléotides dudit type étant aptes à être consommés durant la réplication pour constituer des acides nucléiques répliqués, et ;
**en ce que** lesdits moyens de mesure (42, P) fournissent une valeur décroissante de courant électrique lorsque lesdits moyens d'amplification sont activés, si ledit acide nucléique contient ladite séquence cible déterminée.

## Claims

1. A method for the electrochemical detection of target nucleic acid sequences, said method being of the type according to which:
- a biological sample that may contain at least one nucleic acid is provided, said nucleic acid being capable of containing a given target sequence,
- an oxidizing agent is provided, which is capable of oxidizing at least one nucleotide base of the said target sequence;
- complementary means capable of coupling with said given target sequence are provided, the said complementary means comprising activatable amplification means suitable for replicating said target sequence when the said biological sample is mixed with the said activatable amplification means, said amplification means comprising at least nucleotides of a type which includes said nucleotide base, wherein said nucleotides of said type are able to be consumed during replication so as to constitute replicated nucleic acids
- said oxidizing agent is mixed with said biological sample;
- an electric field is applied to said sample, said electric field being capable of causing a reaction of said oxidizing agent with said nucleotide base, and an electric current which passes through said sample is measured in order to determine the presence of said target sequence if the electric current decreases;
**characterized in that** it comprises, in order, the following steps:
a) said biological sample and said oxidizing agent are mixed with said activatable amplification means;
b) said activatable amplification means are activated; and
c) the biological sample is subjected to an electric field to measure the current which results therefrom in order to determine the presence of said given target sequence if the electric current decreases.

2. The detection method as claimed in claim 1, **characterized in that** said nucleic acid is a DNA or RNA molecule.

3. The detection method as claimed in claim 1 or 2, **characterized in that** said nucleotide base is a purine nitrogenous base.

4. The detection method as claimed in claim 3, **characterized in that** said nucleotide base is guanine or a chemical analog of guanine.

5. The detection method as claimed in any one of claims 1 to 4, **characterized in that** said oxidizing agent is a ruthenium complex.

6. The detection method as claimed in any one of claims 1 to 5, **characterized in that**, since said target sequences are replicated over time, said electric current is measured after having applied said electric field for a given period of time.

7. The detection method as claimed in any one of claims 1 to 6, **characterized in that** said target sequence is replicated according to amplification cycles, and **in that** said current is measured after a given number of cycles.

8. The detection method as claimed in any one of claims 1 to 7, **characterized in that** said electric field is applied between electrodes suitable for being bathed in said sample.

9. The detection method as claimed in claim 8, **characterized in that** one of said electrodes is a metal oxide-based electrode.

10. The detection method as claimed in claim 8, **characterized in that** one of said electrodes is a noble metal-based electrode.

11. The detection method as claimed in claim 8, **characterized in that** one of said electrodes is a carbon electrode.

12. The detection method as claimed in any one of claims 8 to 11, **characterized in that** said oxidizing agent is confined at the surface of one of said electrodes.

13. The detection method as claimed in any one of claims 1 to 12, **characterized in that** it also comprises the following steps:
- a redox compound is also provided, which is capable of reacting at a shifted electric field value different from the electric field value at which said oxidizing agent and said nucleotide base react;
- the value of the redox electric current at said shifted electric field value is measured; and
- the presence of said given target sequence is determined if the difference in electric current between the redox electric current and said electric current of reaction of said oxidizing agent and of said nucleotide base decreases when said amplification means are activated.

14. A collection for the electrochemical detection of target nucleic acid sequences, said collection comprising:
- means (30, 44, 94) for receiving a biological sample (34, 95) that may contain at least one nucleic acid, said nucleic acid being capable of containing a given target sequence, said biological sample being mixed with an oxidizing agent, said target sequence comprising at least one nucleotide base that can be oxidized by said oxidizing agent;
- complementary means capable of coupling with said given target sequence;
- means (38, 42, 52, 54, 96) for applying an electric field to said sample, said electric field being capable of causing a reaction of said oxidizing agent with said nucleotide base, and means for measuring (42, P) an electric current which passes through said sample in order to determine the presence of said target sequence;
**characterized in that** said complementary means comprise activatable amplification means suitable for replicating said target sequence, said amplification means comprising at least nucleotides of a type which includes said nucleotide base, wherein said nucleotides of said type are able to be consumed during replication so as to constitute replicated nucleic acids; and
**in that** said measuring means (42, P) give a decreasing electric current value when said amplification means are activated, if said nucleic acid contains said given target sequence.

## Patentansprüche

1. Verfahren zum elektrochemischen Nachweis von Nucleinsäure-Zielsequenzen, wobei das Verfahren so beschaffen ist, dass man:
- eine biologische Probe bereitstellt, die mindestens eine Nucleinsäure enthalten kann, wobei die Nucleinsäure eine bestimmte Zielsequenz enthalten kann;
- ein Oxidationsmittel bereitstellt, das zur Oxidation mindestens einer Nucleotidbase der bestimmten Zielsequenz befähigt ist;
- komplementäre Mittel bereitstellt, die zur Kupplung mit der bestimmten Zielsequenz geeignet sind, wobei die komplementären Mittel aktivierbare Amplifikationsmittel umfassen, die zur Replikation der Zielsequenz befähigt sind, wenn die biologische Probe mit den aktivierbaren Amplifikationsmitteln vermischt wird, wobei die Amplifikationsmittel mindestens Nuclotide eines Typs, der die Nuclotidbase einschließt, umfassen, wobei die Nucleotide dieses Typs dazu geeignet sind, während der Replikation zur Bildung der replizierten Nucleinsäuren verbraucht zu werden;
- dieses Oxidationsmittel in die biologische Probe einmischt;
- ein elektrisches Feld man die Probe anlegt, wobei das elektrische Feld dazu befähigt ist, eine Reaktion des Oxidationsmittels mit der Nucleotidbase hervorzurufen; und einen elektrischen Strom misst, der die Probe durchfließt, um das Vorliegen der Zielsequenz festzustellen, wenn der elektrische Strom sich verringert;
**dadurch gekennzeichnet, dass** das Verfahren nacheinander die folgenden Stufen umfasst:
(a) man vermischt die biologische Probe und das Oxidationsmittel mit den aktivierbaren Amplifikationsmitteln;
(b) man aktiviert die aktivierbaren Amplifikationsmittel; und
(c) man unterwirft die biologische Probe während der Amplifikation einem elektrischen Feld, um den daraus resultierenden Strom zu messen, so dass die Anwesenheit der bestimmten Zielsequenz festgestellt wird, wenn sich der elektrische Strom verringert.

2. Nachweisverfahren nach Anspruch 1, **dadurch gekennzeichnet, dass** es sich bei der Nucleinsäure um ein DNA- oder RNA-Molekül handelt.

3. Nachweisverfahren nach Anspruch 1 oder 2, **dadurch gekennzeichnet, dass** es sich bei der Nucleotidbase um eine stickstoffhaltige Purinbase handelt.

4. Nachweisverfahren nach Anspruch 3, **dadurch gekennzeichnet, dass** es sich bei der Nucleotidbase um Guanin oder ein chemisches Analoges von Guanin handelt.

5. Nachweisverfahren nach einem der Ansprüche 1 bis 4, **dadurch** gekennzeichet, dass es sich beim Oxidationsmittel um einen Rutheniumkomplex handelt.

6. Nachweisverfahren nach einem der Ansprüche 1 bis 5, **dadurch gekennzeichnet, dass** die Zielsequenzen im Laufe der Zeit repliziert werden und man den elektrischen Strom misst, nachdem das elektrische Feld während einer bestimmten Zeitspanne angelegt worden ist.

7. Nachweisverfahren nach einem der Ansprüche 1 bis 6, **dadurch gekennzeichnet, dass** man die Zielsequenz gemäß den Amplifikationszyklen repliziert und den Strom nach einer bestimmten Unzahl von Zyklen misst.

8. Nachweisverfahren nach einem der Ansprüche 1 bis 7, **dadurch gekennzeichnet, dass** man das elektrische Feld zwischen Elektroden anlegt, die von der Probe gespült werden können.

9. Nachweisverfahren nach Anspruch 8, **dadurch gekennzeichnet, dass** es sich bei einer der Elektroden um eine Elektrode auf der Basis von Metalloxiden handelt.

10. Nachweisverfahren nach Anspruch 8, **dadurch gekennzeichnet, dass** es sich bei einer der Elektroden um eine Elektrode auf der Basis eines Edelmetalls handelt.

11. Nachweisverfahren nach Anspruch 8, **dadurch gekennzeichnet, dass** es sich bei einer der Elektroden um eine Kohlenstoffelektrode handelt.

12. Nachweisverfahren nach einem der Ansprüche 8 bis 11, **dadurch gekennzeichnet, dass** das Oxidationsmittel an die Oberfläche einer der Elektroden angrenzt.

13. Nachweisverfahren nach einem der Ansprüche 1 bis 12, **dadurch gekennzeichnet, dass** es ferner die folgenden Stufen umfasst:
- man stellt ferner eine Redoxverbindung bereit, die dazu befähigt ist, auf einen Wert des verschobenen elektrischen Feldes zu reagieren, der sich vom Wert des elektrischen Feldes unterscheidet, bei dem das Oxidationsmittel und die Nuclotidbase reagieren;
- man misst den Wert des elektrischen Redoxstroms am Wert des verschobenen elektrischen Feldes; und
- man stellt die Anwesenheit der bestimmten Zielsequenz fest, wenn die Differenz des elektrischen Stroms zwischen dem elektrischen Redoxstrom und dem elektrischen Strom aufgrund der Reaktion des Oxidationsmittels und der Nuclotidbase sich verringert, wenn die Amplifikationsmittel aktiviert sind.

14. Vorrichtung zum elektrochemischen Nachweis von Nucleinsäure-Zielsequenzen, wobei die Vorrichtung Folgendes umfasst:
- Mittel (30, 44, 94) zum Aufnehmen einer biologischen Probe (34, 95), die mindestens eine Nucleinsäure enthalten kann, wobei die Nucleinsäure eine bestimmte Zielsequenz enthalten kann, wobei die biologische Probe mit einem Oxidationsmittel vermischt ist, wobei die Zielsequenz mindestens eine Nucleotidbase umfasst, die durch das Oxidationsmittel oxidiert werden kann;
- koplementäre Mittel, die mit der bestimmten Zielsequenz gekuppelt werden können;
- Mittel (38, 42, 52, 54, 96) zum Anlegen eines elektrischen Feldes an die Probe, wobei das elektrische Feld dazu befähigt ist, eine Reaktion des Oxidationsmittels mit der Nucleotidbase hervorzurufen, und eine Messeinrichtung (42, P) für einen elektrischen Strom, der die Probe durchfließt, um die Anwesenheit der Zielsequenz festzustellen;
**dadurch gekennzeichnet, dass** die komplementären Mittel Amaplifikationsmmittel umfassen, die dazu befähigt sind, die Zielsequenz zu replizieren, wobei die Amplifikationsmittel mindestens Nucleotide eines Typs unter Einschluss der Nucleotidbase umfassen, wobei die Nucleotide dieses Typs dazu befähigt sind, während der Replikation verbraucht zu werden, um replizierte Nucleinsäuren zu bilden; und
dass die Messeinrichtung (42, P) dann einen geringeren Wert des elektrischen Stroms liefert, wenn die Amplifikationsmittel aktiviert sind, sofern die Nucleinsäure die bestimmte Zielsequenz enthält.
